# EUROPÄISCHE PATENTSCHRIFT

(11) **EP 1 886 708 B1**
(45) Veröffentlichungstag und Bekanntmachung des Hinweises auf die Patenterteilung: **06.10.2010**
(21) Anmeldenummer: 07108143.4
(22) Anmeldetag: 14.05.2007
(51) Int. Cl.: A61M 21/02, H05B 37/02

(54) **Leuchte mit "Melatonin-schonender" Wirkung**
Lamp with melatonin protection effect
Eclairage à effet augmentant la mélatonine

(30) Priorität: 09.08.2006 DE 102006037222
(43) Veröffentlichungstag der Anmeldung: 13.02.2008
(73) Patentinhaber: Zumtobel Lighting GmbH, 6850 Dornbirn (AT)
(72) Erfinder: Swoboda, Gerald, 6911 Lochau (AT); Schumann, Sabine, 9030 Abtwil (CH)
(74) Vertreter: Thun, Clemens

(56) Entgegenhaltungen:
- EP-A- 1 619 648
- JP-A- 11 135 273
- JP-A- 11 144 510
- JP-A- 2001 149 478
- JP-A- 2003 288 995
- JP-A- 2005 310 654
- JP-A- 2006 252 944
- US-A1- 2006 152 525

## Beschreibung

Die vorliegende Erfindung betrifft ein Verfahren zur Ansteuerung einer Leuchte, mit der Licht in unterschiedlichen spektralen Zusammensetzungen erzeugt werden kann, sowie eine entsprechende Leuchte.

Aus der medizinischen und biologischen Forschung ist bekannt, dass Licht für den Menschen (und allgemein für Säugetiere) nicht nur mit Bezug auf das Sehen von Bedeutung ist, sondern auch anderweitigen Einfluss auf den Organismus hat. Beispielsweise wird durch Licht im Allgemeinen die Epiphyse (Zirbeldrüse) beeinflusst. Die Epiphyse schüttet insbesondere in Abhängigkeit von der Tageszeit und der Helligkeit des auf die Netzhaut eines Menschen auftreffenden Lichtes das Hormon Melatonin aus; auf diese Weise gelangt abhängig von der Tageszeit aber beeinflusst von der Helligkeit mehr oder weniger Malatonin in das Blut des Menschen. Typischerweise dauert es einige Minuten, bis das von der Epiphyse ausgeschüttete Melatonin im Blut verteilt ist.

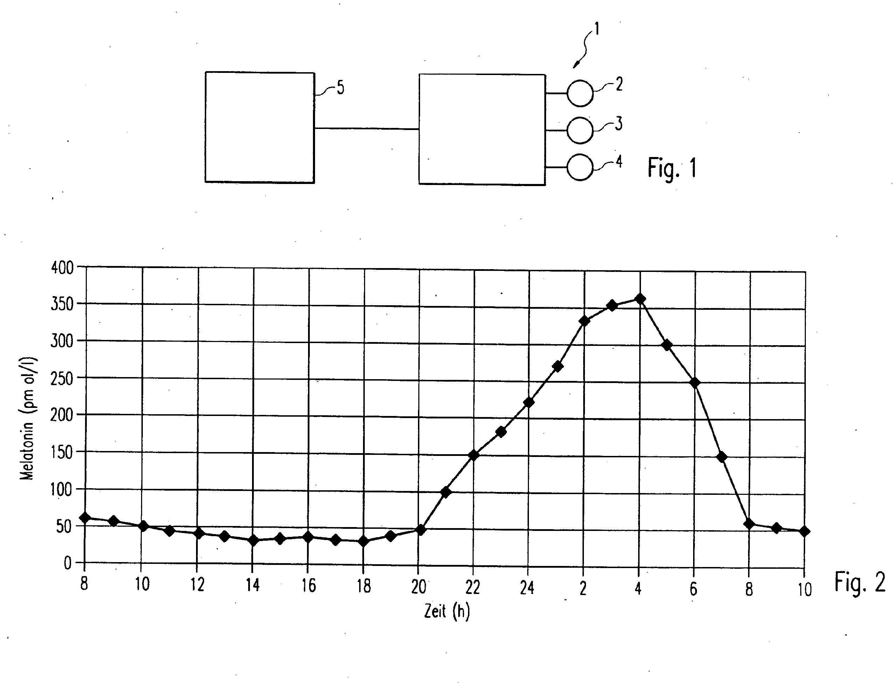

In Fig. 2 ist ein typischer Verlauf des Melatonin-Spiegels im menschlichen Blut in Abhängigkeit von der Tageszeit dargestellt. Man erkennt, dass während der Nacht der Melatonin-Spiegel deutlich höher ist als am Tag. Bei Nacht wird also vergleichsweise viel Melatonin von der Epiphyse ausgeschüttet, bei Tag hingegen sehr wenig.

Das menschliche Gehirn verfügt über einen "internen" (über die rhythmische Aktivität bestimmter Gene gesteuerten) circadianen Taktgeber, den so genannten Nucleus suprachiasmaticus, der über optische Nervenfasern mit der Netzhaut verbunden ist. Unter natürlichen Bedingungen wird die Schwingungsdauer dieses Taktgebers durch Licht, das auf die Netzhaut trifft, fortwährend an den natürlichen Tagesrhythmus angeglichen. Der Nucleus suprachiasmaticus sendet - unter anderem - Impulse an die Epiphyse, die daraufhin über die Ausschüttung von Melatonin auf hormonellem Wege Systemen des menschlichen Organismus den circadianen Rhythmus aufprägt.

Aus neueren Untersuchungen ist bekannt, dass die Epiphyse ihre Aktivität nicht nur auf Grund der Helligkeit des auf die Netzhaut auftreffenden Lichtes verändert, sondern dass dabei auch die spektrale Zusammensetzung des Lichtes eine wichtige Rolle spielt.

Das Spektrum bzw. die Farbtemperatur des natürlichen Tageslichtes ändert sich im Allgemeinen im Laufe eines Tages. In der Mittagszeit, bei hohen Beleuchtungsstärken, weist das Tageslicht eine vergleichsweise hohe Farbtemperatur auf, somit einen vergleichsweise hohen Anteil im blauen Bereich des Spektrums, während in der Dämmerung oder bei Sonnenauf- oder -untergang das natürliche Licht eine niedrige Farbtemperatur und somit einen vergleichsweise geringen Blau-Anteil aufweist.

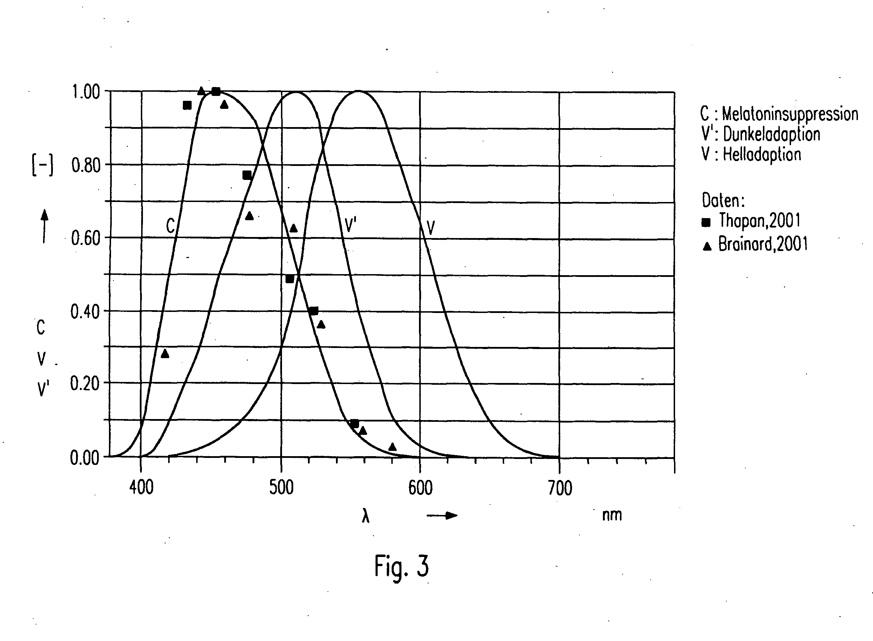

Die letztgenannten neueren Untersuchungen haben nun gezeigt, dass der blaue Anteil des Lichtes die Ausschüttung von Melatonin im Allgemeinen wesentlich mehr unterdrücken kann als der rote Anteil des Lichtes (bei gleicher Intensität). In Fig. 3 ist diese Abhängigkeit näher dargestellt. Mit der Kurve "C" ist das empirisch ermittelte Aktionsspektrum für die Unterdrückung der Melatoninausschüttung, also für die "Melatoninsuppression" angegeben. Die quadratischen Symbole geben entsprechende Daten nach Thapan, 2001 wieder, die dreieckigen Symbole entsprechende Daten nach Brainard, 2001. Zum Vergleich ist in dem Diagramm auch die Empfindlichkeit des menschlichen Auges für Nachtsehen (Kurve "V'") und Tagsehen (Kurve "V") eingezeichnet. Man erkennt für die Melatoninsuppression ein Wellenlängenmaximum im Bereich von etwa 480 nm, genauer nach Brainard 464 nm bzw. nach Taphan 468 nm, also im blauen Bereich des Spektrums, und eine deutliche Abnahme zwischen etwa 520 nm und 560 nm. Strahlung mit Wellenlängen von mehr als ca. 560 nm hat demnach nur sehr geringes Potenzial zur Unterdrückung der Melatonin-Ausschüttung.

Wenn ein Mensch nachts Kunstlicht ausgesetzt wird, besteht im Allgemeinen die Möglichkeit, dass die Melatonin-Ausschüttung dieses Menschen durch das Kunstlicht negativ beeinflusst wird, insbesondere gehemmt wird, so dass die Melatonin-Abgabe in das Blut bei diesem Menschen entsprechend reduziert wird. Als Folge hiervon sind unerwünschte Auswirkungen, wie beispielsweise eine Reduzierung der Schlafqualität oder gar eine Schwächung des Immunsystems nicht auszuschließen. Dabei ist zu erwarten, dass der unerwünschte Effekt umso deutlicher ausgeprägt ist, je intensiver das Licht ist, da die Melatoninsuppression mit zunehmender Helligkeit ansteigt. Weiterhin ist davon auszugehen, dass der unerwünschte Effekt umso größer ist, je länger der Mensch dem Licht nachts ausgesetzt ist.

Es sei an dieser Stelle angemerkt, dass nach heutigem Stand (Juli 2006) die Forschungen hinsichtlich der Melatonin-Wirkung keineswegs als abgeschlossen angesehen werden können. Es ist davon auszugehen, dass in Zukunft noch weitere wichtige Erkenntnisse in diesem Zusammenhang gewonnen werden können, beispielsweise mit Bezug auf das oben in Verbindung mit Fig. 3 dargestellte Aktionspotenzial der Melatoninsuppression.

Mit Bezug auf Leuchten, die Licht in unterschiedlichen spektralen Zusammensetzungen abgeben können, sind aus dem Stand der Technik so genannte "RGB-Leuchten" bekannt, die über drei Lichtquellen verfügen, die Licht in unterschiedlichen Farben oder Farbtemperaturen ausstrahlen können. Die Farben der Lichtquellen sind üblicherweise rot (R), gelb (G) und blau (B). Durch Mischung von Licht der drei Lichtquellen kann mit einer derartigen Leuchte Licht in unterschiedlichen Farben erzeugt werden. Beispielsweise sind derartige Leuchten bekannt, mit denen durch entsprechende Licht-Mischungen zu bestimmten Zeiten bestimmte Effekte bzw. Wirkungen erzielt werden sollen.

Weiterhin sind aus dem Stand der Technik Leuchten bekannt, die Licht mit einem Spektrum aussenden, das dem natürlichen Lichtspektrum nachgebildet ist. Ziel derartiger Leuchten ist unter anderem, tagsüber die Ausschüttung von Melatonin zu hemmen, beispielsweise um winterlichen Depressionen, die durch Lichtmangel verursacht sind, entgegenzuwirken.

Aus der EP 1 577 163 A2 ist eine Bestrahlungsvorrichtung zur Steigerung der Vigilanz eines Fahrers eines Fahrzeugs bekannt. Dazu ist im Kopfbereich des Fahrers eine Leuchte zur Unterdrückung der Melatoninproduktion und Erhöhung der Wachsamkeit vorgesehen.

Aus der EP 1 619 648 A1 ist eine Bildschirmeinheit bekannt, bei der nachts Licht mit reduziertem Blau-Anteil abgegeben wird und tagsüber Licht mit erhöhtem Blau-Anteil; hierdurch kann auf den Bio-Rhythmus des Betrachters eingewirkt werden. Dabei kann vorgesehen sein, dass bei zunehmender Intensität des abgegebenen Lichts der Blau-Anteil nicht proportional zum Gesamtlicht zunimmt.

Aus dem japanischen Patentdokument JP 11-144510 ist eine Leuchte bekannt, mit der zu bestimmten Zeiten unterschiedliche Lichtarten mit unterschiedlichen Farbtemperaturen erzeugt werden können, so dass sich unterschiedliche Stimmungen hervorrufen lassen. Aus dem Dokument JP 11-135273 ist eine Leuchte bekannt, mit der Licht erzeugt werden kann, das in alternierender Form wechselnde Farbtemperaturen aufweist, beispielsweise zwischen 3000 K und 6700 K.

Vor diesem Hintergrund kann es als Aufgabe der vorliegenden Erfindung angesehen werden, ein Verfahren zur Ansteuerung einer Leuchte bzw. eine entsprechende Leuchte anzugeben, mit dem bzw. mit der einer möglichen negativen Auswirkung mit Bezug auf die circadiane Rhythmik eines Betrachters entgegen gewirkt werden kann und/oder eine bestimmte circadiane Rhythmik unterstützt werden kann.

Diese Aufgabe wird mit den Gegenständen der unabhängigen Ansprüche gelöst. Vorteilhafte Ausgestaltungen sind in den abhängigen Ansprüchen angegeben.

Gemäß einem ersten Aspekt der Erfindung ist ein Verfahren zur Ansteuerung einer Leuchte vorgesehen, mit der Licht in unterschiedlichen spektralen Zusammensetzungen erzeugt werden kann. Dabei wird bei der Ansteuerung die spektrale Zusammensetzung des erzeugten Lichtes in Abhängigkeit von einem vorgegebenen Zeitschema gewählt. Erfindungsgemäß wird dabei in einem vorab definierten Zeitraum ein besonderes Licht erzeugt, das hinsichtlich seiner spektralen Zusammensetzung und Intensität derart ist, dass es bei einem Betrachter des besonderen Lichtes eine Ausschüttung des Hormons Melatonin zumindest weitestgehend nicht hemmt.

Auf diese Weise kann mit Hilfe einer entsprechenden Wahl des vorab definierten Zeitraums eine Beleuchtung zur Verfügung gestellt werden, mit der einer möglichen negativen Auswirkung mit Bezug auf die circadiane Rhythmik des Menschen entgegen gewirkt werden kann bzw. eine bestimmte circadiane Rhythmik unterstützt werden kann.

Mit der Formulierung "Betrachter des besonderen Lichtes" soll zum Ausdruck gebracht werden, dass der Betrachter zumindest im Wesentlichen kein Licht betrachtet, das nicht von der Leuchte erzeugt wird. Der fiktive Betrachter wird also sozusagen zumindest im Wesentlichen ausschließlich einem Licht ausgesetzt, das von der Leuchte erzeugt wird.

Es kann vorgesehen sein, dass das besondere Licht zumindest im Wesentlichen nur indirekt auf die Netzhaut des Betrachters trifft, also der Betrachter normalerweise nicht direkt in die Leuchte blickt. Beispielsweise kann eine Leuchte vorgesehen sein, die sich an einer Decke eines Raumes befindet, in dem sich der Betrachter aufhält, und zwar derart, dass der Betrachter nicht direkt in die Leuchte blickt, sofern er den Kopf nicht nach oben wendet.

Insbesondere kann vorgesehen sein, dass bei dem Betrachter auch dann die Melatonin-Aussschüttung nicht gehemmt wird, wenn er dem besonderen Licht für einen bestimmten längeren Zeitraum ausgesetzt ist, beispielsweise für einen Zeitraum von wenigstens 30 Minuten oder wenigstens zwei Stunden.

Als vorab festgelegter Zeitraum können insbesondere mehrere, voneinander beabstandete Zeitintervalle festgelegt sein, beispielsweise "nächtliche" Zeiten.

Zur Festlegung des vorab definierten Zeitraums können die Zeiten von Sonnenaufgängen und/oder Sonnenuntergängen genutzt werden. Dabei kann der Ort zu Grunde gelegt werden, an dem der Betrieb der Leuchte vorgesehen ist. Es kann also beispielsweise vorgesehen sein, als vorab definierten Zeitraum die Zeitintervalle zwischen Sonnenuntergängen und den jeweils darauf folgenden Sonnenaufgängen zu wählen.

Es kann jedoch mit Bezug auf die Zeiten der Sonnenaufgänge und Sonnenuntergänge auch ein Ort oder mehrere Orte gewählt werden, der bzw. die nicht der Ort des Betriebs der Leuchte ist bzw. sind. Dies wäre zum Beispiel sinnvoll, wenn eine Verschiebung der circadianen Rhythmik bei dem Betrachter erwünscht ist, beispielsweise um einem Jetlag vorzubeugen oder um die Symptome eines Jetlags zu mildern. In diesem Fall kann es weiterhin von Vorteil sein, wenn als Zeitraum voneinander beabstandete Zeitintervalle gewählt werden, die dementsprechend länger oder kürzer als 24 Stunden sind.

Mit Bezug auf die spektrale Zusammensetzung des besonderen Lichts ist es von Vorteil, wenn derjenige Anteil an der Intensität des besonderen Lichtes, der durch Strahlung mit Wellenlängen erzeugt wird, die kleiner sind als ein vorgegebener Wellenlängen-Schwellenwert, kleiner ist als ein vorgegebener Anteil-Schwellenwert.

Beispielsweise kann der Wellenlängen-Schwellenwert dabei etwa zwischen 480 nm und 600 nm liegen, vorzugsweise zwischen etwa 500 nm und 560 nm. Dies ist vorteilhaft, wenn man das oben in Verbindung mit Fig. 3 dargestellte Aktionsspektrum für die Unterdrückung der Melatoninausschüttung zu Grunde legt. Es ist jedoch zu bedenken, dass sich in diesem Zusammenhang auf Grund zukünftiger Erkenntnisse entsprechende Abweichungen von diesen Bereichen ergeben können. Allgemeiner formuliert ist es also vorteilhaft, wenn der Wellenlängen-Schwellenwert in Abhängigkeit des Aktionsspektrums für die Unterdrückung der Melatoninausschüttung festgelegt wird.

Es ist weiterhin vorteilhaft, wenn der Anteil-Schwellenwert kleiner als 10% gewählt wird, vorzugsweise kleiner als 5%. Selbstverständlich kann für den Anteil-Schwellenwert auch 0% gewählt werden. Es ist jedoch in diesem Zusammenhang zu bedenken, dass für die Güte der Farbwahrnehmung ein gewisser Anteil an blauem Licht durchaus von Vorteil ist. Daher kann ein Kompromiss vorgesehen sein, der darin besteht, für das besondere Licht einen gewissen, allerdings geringen, Anteil an blauem Licht vorzusehen, beispielsweise kann also vorgesehen sein, dass das besondere Licht außerdem für den genannten Anteil einen vorab festgelegten Anteil-Mindestwert aufweist, der beispielsweise etwa zwischen 1% und 5% betragen kann.

Es ist von Vorteil, wenn das besondere Licht eine Farbtemperatur aufweist, die unterhalb eines vorab festgelegten Farbtemperatur-Schwellenwertes liegt. Beispielsweise kann als entsprechender Schwellenwert 2000 K, vorzugsweise 1500 K oder noch weniger vorgesehen sein.

Weiterhin ist es von Vorteil, wenn bei der Ansteuerung die spektrale Zusammensetzung des erzeugten Lichtes nicht nur in Abhängigkeit von dem vorab definierten Zeitraum, sondern auch in Abhängigkeit von der Intensität des besonderen Lichtes gewählt wird. Insbesondere kann dabei vorgesehen sein, dass der Anteil an der Intensität des besonderen Lichtes, der durch Strahlung mit Wellenlängen erzeugt wird, die kleiner sind als der vorgegebene Wellenlängen-Schwellenwert (kurz "Blau-Anteil"), im Fall einer ersten Intensität des besonderen Lichtes kleiner ist als im Fall einer zweiten Intensität des besonderen Lichtes, die kleiner ist als die erste Intensität des besonderen Lichtes.

Hierdurch wird ermöglicht, dass bei einer kleinen Intensität des besonderen Lichtes ein "Blau-Anteil" gewählt wird, der so groß ist, dass Farben sehr gut unterschieden werden können. Bei einer größeren Intensität des besonderen Lichtes, bei der ein entsprechender Blau-Anteil bereits eine Unterdrückung der Melatonin-Ausschüttung unerwünscht hemmen könnte, kann der Blau-Anteil entsprechend reduziert werden. Auf diese Weise kann bei entsprechend geringen Intensitäten gewährleistet werden, dass die Melatonin-Ausschüttung nicht gehemmt wird und dennoch Farben gut unterschieden werden können. Beispielsweise könnte vorgesehen sein, dass bei einer Intensität des besonderen Lichtes, die über einem Intensitäts-Schwellenwert liegt, der Blau-Anteil auf weniger als 1% reduziert wird. Als Schwellenwert für die Intensität könnte beispielsweise eine Stärke des besonderen Lichtes von etwa zwischen 100 lux und 500 lux, je nach spektraler Zusammensetzung, dienen.

Bei der verwendeten Leuchte kann es sich um eine Leuchte mit drei Lichtquellen handeln, vorzugsweise in Form von Leuchtstofflampen, die jeweils Licht in einer anderen Farbe oder Farbtemperatur erzeugen können. Vorteilhaft ist es, wenn dabei das besondere Licht von (nur) einer der drei Lichtquellen erzeugt werden kann. Beispielsweise kann es sich bei der Leuchte um eine RGB-Leuchte handeln.

Gemäß einem weiteren Aspekt der Erfindung ist eine Leuchte vorgesehen, die dazu ausgebildet ist, Licht in unterschiedlichen spektralen Zusammensetzungen abzugeben. Die Leuchte weist dabei wenigstens zwei Lichtquellen und eine Steuereinrichtung für die Lichtquellen auf. Erfindungsgemäß ist dabei die Steuereinrichtung dazu ausgebildet, die Leuchte derart anzusteuern, dass von der Leuchte in einem vorab definierten Zeitraum ein besonderes Licht abgegeben wird, das hinsichtlich seiner spektralen Zusammensetzung und Intensität derart ist, dass es bei einem Betrachter des besonderen Lichtes eine Ausschüttung des Hormons Melatonin zumindest weitestgehend nicht hemmt.

Es ist von Vorteil, wenn das besondere Licht von einer der Lichtquellen erzeugt wird.

Die Lichtquellen können vorteilhaft Leuchtstofflampen umfassen.

Es ist von Vorteil, wenn die Leuchte drei Lichtquellen aufweist, die Licht in unterschiedlichen Farben oder Farbtemperaturen ausstrahlen können. Dabei kann die Leuchte drei Steuerungskanäle für die drei Lichtquellen aufweisen.

Weiterhin kann vorgesehen sein, dass in diesem Fall mit einer ersten der drei Lichtquellen Licht mit einer Farbtemperatur von mehr als 5000 K ausgestrahlt werden kann, und mit einer zweiten der drei Lichtquellen Licht mit einer Farbtemperatur von weniger als 5000 K, und mit der dritten Lichtquelle das besondere Licht.

Vorteilhaft gilt für die spektrale Zusammensetzung des besonderen Lichtes Folgendes: derjenige Anteil an der Intensität des besonderen Lichtes, der durch Strahlung mit Wellenlängen erzeugt wird, die kleiner sind als ein vorgegebener Wellenlängen-Schwellenwert, ist so klein, dass er unterhalb eines vorgegebenen Anteil-Schwellenwertes liegt. Dabei kann der Wellenlängen-Schwellenwert etwa zwischen 480 nm und 600 nm liegen, vorzugsweise zwischen 500 nm und 560 nm. Der Anteil-Schwellenwert kann kleiner als 10% gewählt sein, vorzugsweise kleiner als 5%.

Vorteilhaft weist das besondere Licht eine Farbtemperatur auf, die unterhalb eines vorab festgelegten Farbtemperatur-Schwellenwertes liegt. Dieser Schwellenwert kann dabei maximal etwa 2000 K, vorzugsweise weniger als 1500 K betragen.

Die Leuchte kann vorteilhaft weiterhin dazu ausgebildet sein, Licht in unterschiedlichen Intensitäten abzugeben, wobei die Steuereinrichtung weiterhin dazu ausgebildet ist, die Leuchte derart anzusteuern, dass bei der Ansteuerung die spektrale Zusammensetzung des besonderen Lichtes (auch) in Abhängigkeit von der Intensität des besonderen Lichtes gewählt wird. Dabei kann vorgesehen sein, dass die Steuereinrichtung dazu ausgebildet ist, die Leuchte derart anzusteuern, dass für das besondere Licht gilt: der Anteil an der Intensität des besonderen Lichtes, der durch Strahlung mit Wellenlängen erzeugt wird, die kleiner sind als der vorgegebene Wellenlängen-Schwellenwert, ist im Fall einer ersten Intensität des besonderen Lichtes kleiner ist als im Fall einer zweiten Intensität des besonderen Lichtes, die kleiner ist als die erste Intensität des besonderen Lichtes.

Die Erfindung wir im Folgenden an Hand eines Ausführungsbeispiels und mit Bezug auf die Zeichnungen näher erläutert. Es zeigen:
- Fig. 1: eine schematische Übersicht über Komponenten einer erfindungsgemäßen Leuchte,
- Fig. 2: ein Diagramm zum typischen Verlauf des Melatonin-Spiegels in Abhängigkeit von der Tageszeit,
- Fig. 3: ein Diagramm zur Suppression der Melatoninausschüttung in Abhängigkeit der Wellenlänge des auf die Netzhaut fallenden Lichtes, und
- Fig. 4: die Normfarbtafel nach CIE mit der Plank'schen Kurve.

In Fig. 1 sind wesentliche Komponenten einer erfindungsgemäßen Leuchte 1 dargestellt. Die Leuchte 1 umfasst gemäß diesem Ausführungsbeispiel drei Lichtquellen 2, 3, 4, beispielsweise in Form von jeweils mehreren Leuchtstofflampen, die Licht in drei unterschiedlichen Farbtemperaturen abgeben können. Beispielsweise kann vorgesehen sein, dass die erste Lichtquelle 2 Licht möglichst hoher Farbtemperatur, beispielsweise etwa 8000 K abgeben kann, und die zweite Lichtquelle "warmweißes" Licht, beispielsweise mit einer Farbtemperatur von etwa 2700 K. Die dritte Lichtquelle 4 kann ein besonderes Licht abgeben, das im Folgenden als "Melatonin-Licht" bezeichnet wird.

Dabei ist das Melatonin-Licht hinsichtlich seiner spektralen Zusammensetzung und Intensität derart gewählt, dass es bei einem Betrachter eine Ausschüttung des Hormons Melatonin zumindest weitestgehend nicht hemmt. Auf die Eigenschaften des Melatonin-Lichtes wird weiter unten näher eingegangen.

Weiterhin weist die Leuchte 1 eine Steuereinrichtung 5 auf, mit der die drei Lichtquellen 2, 3, 4 angesteuert werden können. Die Steuereinrichtung 5 verfügt über drei Steuerungskanäle, mit deren Hilfe die drei Lichtquellen 2, 3, 4 unabhängig voneinander, vergleichbar zu den Verhältnissen bei einer RGB-Leuchte, angesteuert werden können. Die Intensitäten der drei Lichtquellen 2, 3, 4 sind also unabhängig voneinander einstellbar.

Für die Ansteuerung der Leuchte wird vorab ein Zeitraum definiert, wobei gemäß diesem Ausführungsbeispiel hierfür nächtliche Zeitintervalle mit Hilfe der Zeitpunkte von Sonnenauf- und -untergängen festgelegt werden. Der Zeitraum umfasst also "nächtliche Zeiten".

Für die Ansteuerung des Melatonin-Lichtes gilt nun Folgendes: Wenn innerhalb des festgelegten Zeitraums, hier also "nachts", die Leuchte 1 eingeschaltet wird, wird das Melatonin-Licht von der Leuchte 1 abgestrahlt. Wenn in einem Zeitpunkt, der nicht in dem definierten Zeitraum liegt, hier also "tagsüber" die Leuchte 1 eingeschaltet wird, wird "normales" Licht von der Leuchte 1 abgestrahlt. Weiterhin kann vorgesehen sein, dass in dem Fall, in dem die Leuchte 1 innerhalb des definierten Zeitraums, hier also "nachts", eingeschaltet wird und so lange angeschaltet bleibt, bis der Zeitraum verlassen wird, hier also bis zum folgenden "Tag", zum Zeitpunkt des Verlassens, also "morgens" automatisch eine (beispielsweise fließende) Änderung von Melatonin-Licht in "normales" Licht erfolgt. Entsprechendes kann für den Übergang von "Tag" zu "Nacht" vorgesehen sein.

Für das "normale" Licht kann dabei weiterhin vorgesehen sein, dass ein Licht gewählt wird, das bei einem Betrachter eine aktivierende Stimmung auslöst, also beispielsweise ein Licht einer bestimmten Mindesthelligkeit, beispielsweise im Bereich von wenigstens 1000 lux bzw. ein Licht dessen Blau-Anteil einen bestimmten Mindestwert übersteigt.

Das Melatonin-Licht ist also für die Steuereinrichtung abgespeichert. Die circadiane Kurve ist steuerungstechnisch hinterlegt und definiert abhängig von der Tageszeit, wann welches Licht von der Leuchte 1 abgegeben wird.

Das Melatonin-Licht weist eine besondere spektrale Zusammensatzung auf, die beispielsweise mit Hilfe desjenigen Anteils an der Intensität des Melatonin-Lichtes festgelegt werden kann, der durch Strahlung mit Wellenlängen erzeugt wird, die kleiner sind als ein vorgegebener Wellenlängen-Schwellenwert, kurz "Blau-Anteil" A_{BLAU} genannt. Gemäß einem ersten Beispiel ist dieser Blau-Anteil kleiner als ein vorgegebener Anteil-Schwellenwert A_{S}. Aus der Forschung ist bekannt, dass der blaue Anteil des Spektrums eines Lichtes weitaus mehr Potenzial zur Hemmung der Melatonin-Ausschüttung eines Betrachters dieses Lichtes hat, als der restliche Teil des Spektrums, wobei die genauen spektralen Übergänge fließend sind. Im hier dargestellten Ausführungsbeispiel kann als Wellenlängen-Schwellenwert 550 nm gewählt sein und für den Anteil-Schwellenwert 5%.

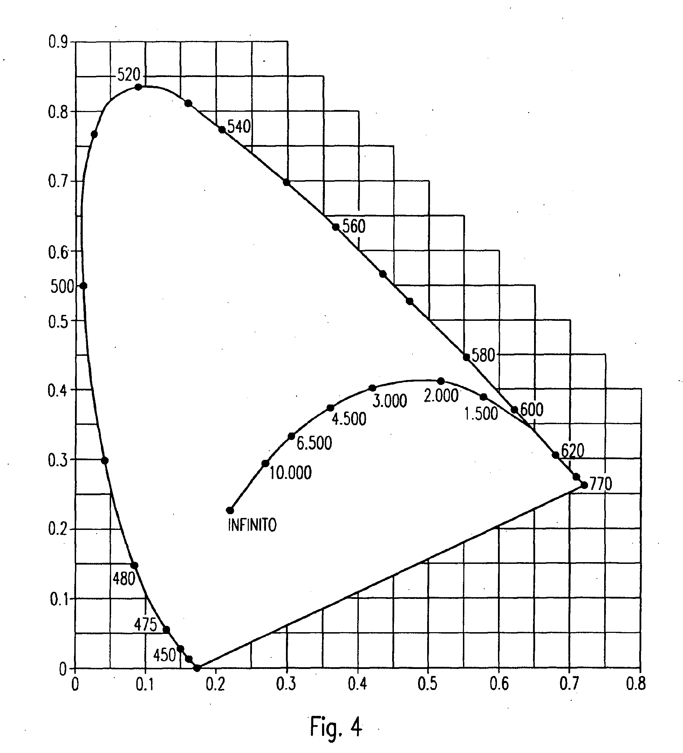

Alternativ ist es möglich, die spektrale Zusammensetzung des Melatonin-Lichtes mit Hilfe der Farbtemperatur festzulegen. Beispielsweise kann für das Melatonin-Licht eine Farbtemperatur von weniger als 1500 K gewählt sein. Zur Orientierung ist in Fig. 4 das bekannte Farbdiagramm gemäß der CIE (CIE: Commission Internationale de L'Eclairage) dargestellt und darin der Planck'sche Kurvenzug für die Schwarzkörper-Strahlung eingetragen. Auf dem Kurvenzug sind zur Orientierung exemplarisch verschiedene (Farb-)Orte eingetragen, die verschiedenen Farbtemperaturen zugeordnet sind. Dabei sind die entsprechenden Werte der Farbtemperaturen in der Einheit Kelvin eingetragen.

Zur technischen Realisierung des Melatonin-Lichtes ist bei diesem Ausführungsbeispiel eine besondere Lampe oder eine entsprechende Kombination aus einer Lampe und einem Filter vorgesehen. Als Filter kann ein Kunststoffschlauch dienen, der über die Lampe gezogen ist und der die Farbe bzw. das Spektrum des abgegebenen Lichts entsprechend verändert.

Bei der Abstimmung der Lampe und gegebenenfalls des Filters ist es vorteilhaft, die Wirkung des Gesamtsystems zu berücksichtigen. Hierzu können alle Teile der Leuchte Berücksichtigung finden, die mit dem Licht interagieren, wie beispielsweise etwaige Reflektoren, Glasabdeckungen, Kunststoffteile und dergleichen. Derartige Teile können das Spektrum bzw. die Farbe oder Farbtemperatur des abgegebenen Lichts beeinflussen.

Als Variante kann vorgesehen sein, dass das Melatonin-Licht zumindest in bestimmten Grenzen dimmbar ist, wobei oberhalb einer bestimmten Helligkeitsgrenze ein Licht mit den oben genannten spektralen Eigenschaften des Melatonin-Lichtes erzeugt wird und unterhalb der Helligkeitsgrenze ein "normales" Spektrum, also insbesondere ein Spektrum mit einem nennenswerten Blau-Anteil. Dadurch kann sichergestellt werden, dass bei vergleichsweise sehr niedrigen Intensitäten des Melatonin-Lichtes von einem Betrachter Farben besser unterschieden werden können, was insbesondere eine Orientierung erleichtert.

Zur Kalibrierung der Leuchte kann vorgesehen sein, dass eine Modulation des Lichtes in dem Raum, in dem die Leuchte betrieben wird, berücksichtigt wird. Eine derartige Modulation findet im Allgemeinen an Oberflächen, beispielsweise von Wänden usw. statt, wobei diese Modulation von dem Material der Oberflächen und deren optischen Eigenschaften abhängt. Um das so entstehende "Raumlicht" im Sinne einer Kalibrierung einzustellen, kann vorgesehen sein, durch Zumischen von Licht weiterer Lampen das Spektrum des Melatonin-Lichtes zu verändern und an den entsprechenden Raum anzupassen. Ziel dieser Kalibrierung kann ein Kompromiss zwischen "Melatonin-schondener" Wirkung einerseits und guter Farbwiedergabe andererseits sein.

Die hier dargestellte Technologie ermöglicht eine vergleichsweise einfache Anpassung der zeitlichen Steuerungskurve und des Spektrums des Melatonin-Lichtes, wie sie durch entsprechende zukünftige neue wissenschaftliche Erkenntnisse erforderlich werden könnte. Beispielsweise könnte eine Anpassung durch entsprechende Auswahl der Leuchtmittel und/oder Filter erfolgen.

Schließlich besteht die Möglichkeit, mit der Leuchte spezielle Anforderungen mit Bezug auf den Betrachter zu berücksichtigen. Beispielsweise könnte bei einem Einsatz in einem Pflegeheim berücksichtigt werden, dass die Transmission der Augenlinse des Betrachters altersbedingt verändert ist. Auch kann die Nutzung der Erfindung in Einrichtungen sinnvoll sein, die mehrere oder viele zu beleuchtende Räume aufweisen, wie beispielsweise Hotels, Krankenhäuser, Altersheime und dergleichen. Beispielsweise kann hierbei vorgesehen sein, dass zur Beleuchtung der Räume erfindungsgemäße Leuchten vorgesehen sind, die mit Hilfe einer zentralen Ansteuerung gesteuert werden können.

### Bezugszeichenliste

- 1: Leuchte
- 2: erste Leuchtstofflampe
- 3: zweite Leuchtstofflampe
- 4: dritte Leuchtstofflampe
- 5: Steureinrichtung

## Patentansprüche

1. Verfahren zur Ansteuerung einer Leuchte, mit der Licht in unterschiedlichen spektralen Zusammensetzungen erzeugt werden kann,
wobei bei der Ansteuerung die spektrale Zusammensetzung des erzeugten Lichtes in Abhängigkeit von einem vorgegebenen Zeitschema gewählt wird, wobei in einem vorab definierten Zeitraum ein besonderes Licht erzeugt wird, das hinsichtlich seiner spektralen Zusammensetzung und Intensität derart ist, dass es bei einem Betrachter des besonderen Lichtes eine Ausschüttung des Hormons Melatonin zumindest weitestgehend nicht hemmt,
bei dem das besondere Licht eine spektrale Zusammensetzung aufweist, für die gilt:
derjenige Anteil (A_{BLAU}) an der Intensität des besonderen Lichtes, der durch Strahlung mit Wellenlängen erzeugt wird, die kleiner sind als ein vorgegebener Wellenlängen-Schwellenwert, ist kleiner als ein vorgegebener Anteil-Schwellenwert (A_{S}),
wobei bei der Ansteuerung der Leuchte die spektrale Zusammensetzung des besonderen Lichtes außerdem in Abhängigkeit von der Intensität des besonderen Lichtes gewählt wird,
**dadurch gekennzeichnet,**
**dass** der Anteil (A_{BLAU}) an der Intensität des besonderen Lichtes, der durch Strahlung mit Wellenlängen erzeugt wird, die kleiner sind als der vorgegebene Wellenlängen-Schwellenwert, im Fall einer ersten Intensität des besonderen Lichtes kleiner ist als im Fall einer zweiten Intensität des besonderen Lichtes, die kleiner ist als die erste Intensität des besonderen Lichtes.

2. Verfahren nach Anspruch 1,
wobei zur Festlegung des vorab definierten Zeitraums die Zeiten von Sonnuntergängen und/oder Sonnenaufgängen genutzt werden.

3. Verfahren nach Anspruch 1 oder 2,
wobei der Wellenlängen-Schwellenwert etwa zwischen 480 nm und 600 nm liegt, vorzugsweise zwischen 500 nm und 560 nm.

4. Verfahren nach einem der vorhergehenden Ansprüche,
wobei der Anteil-Schwellenwert (A_{S}) kleiner als 10% gewählt wird, vorzugsweise kleiner als 5%.

5. Verfahren nach einem der vorhergehenden Ansprüche,
bei dem das besondere Licht eine Farbtemperatur aufweist, die unterhalb eines vorab festgelegten Farbtemperatur-Schwellenwertes liegt.

6. Verfahren nach Anspruch 5,
wobei der Farbtemperatur-Schwellenwert maximal etwa 2000 K, vorzugsweise weniger als 1500 K beträgt.

7. Verfahren nach einem der vorhergehenden Ansprüche,
wobei als Leuchte (1) eine Leuchte mit drei Lichtquellen (2, 3, 4), vorzugsweise in Form von Leuchtstofflampen, verwendet wird, die jeweils Licht in einer anderen Farbe oder Farbtemperatur erzeugen können, wobei das besondere Licht von einer der drei Lichtquellen (2, 3, 4) erzeugt werden kann.

8. Verfahren nach einem der vorhergehenden Ansprüche,
wobei die Leuchte eine RGB-Leuchte ist.

9. Leuchte, die dazu ausgebildet ist, Licht in unterschiedlichen spektralen Zusammensetzungen abzugeben, aufweisend
- wenigstens zwei Lichtquellen, und
- eine Steuereinrichtung (5) für die Lichtquellen,
wobei die Steuereinrichtung (5) dazu ausgebildet ist, die Leuchte (1) derart anzusteuern, dass von der Leuchte (1) in einem vorab definierten Zeitraum ein besonderes Licht abgegeben wird, das hinsichtlich seiner spektralen Zusammensetzung und Intensität derart ist, dass es bei einem Betrachter des besonderen Lichtes eine Ausschüttung des Hormons Melatonin zumindest weitestgehend nicht hemmt,
wobei das besondere Licht eine spektrale Zusammensetzung aufweist, für die gilt:
derjenige Anteil (A_{BLAU}) an der Intensität des besonderen Lichtes, der durch Strahlung mit Wellenlängen erzeugt wird, die kleiner sind als ein vorgegebener Wellenlängen-Schwellenwert, ist so klein, dass er unterhalb eines vorgegebenen Anteil-Schwellenwertes (A_{S}) liegt,
wobei die Leuchte weiterhin dazu ausgebildet ist, Licht in unterschiedlichen Intensitäten abzugeben,
wobei die Steuereinrichtung (5) weiterhin dazu ausgebildet ist, die Leuchte (1) derart anzusteuern, dass bei der Ansteuerung die spektrale Zusammensetzung des besonderen Lichtes in Abhängigkeit von der Intensität des besonderen Lichtes gewählt wird,
**dadurch gekennzeichnet,**
**dass** die Steuereinrichtung (5) dazu ausgebildet ist, die Leuchte (1) derart anzusteuern, dass für das besondere Licht gilt:
der Anteil (A_{BLAU}) an der Intensität des besonderen Lichtes, der durch Strahlung mit Wellenlängen erzeugt wird, die kleiner sind als der vorgegebene Wellenlängen-Schwellenwert, ist im Fall einer ersten Intensität des besonderen Lichtes kleiner ist als im Fall einer zweiten Intensität des besonderen Lichtes, die kleiner ist als die erste Intensität des besonderen Lichtes.

10. Leuchte nach Anspruch 9
bei der das besondere Licht von einer der Lichtquellen erzeugt wird.

11. Leuchte nach Anspruch 9 oder 10,
bei der die Lichtquellen Leuchtstofflampen umfassen.

12. Leuchte nach einem der Ansprüche 9 bis 11,
wobei die Leuchte drei Lichtquellen (2, 3, 4) aufweist, die Licht in unterschiedlichen Farben oder Farbtemperaturen ausstrahlen können.

13. Leuchte nach Anspruch 12,
aufweisend drei Steuerungskanäle für die drei Lichtquellen (2, 3, 4).

14. Leuchte nach Anspruch 12 oder 13,
wobei mit einer ersten (2) der drei Lichtquellen (2, 3, 4) Licht mit einer Farbtemperatur von mehr als 5000 K ausgestrahlt werden kann, und mit einer zweiten (3) der drei Lichtquellen (2, 3, 4) Licht mit einer Farbtemperatur von weniger als 5000 K, und mit der dritten Lichtquelle (4) das besondere Licht.

15. Leuchte nach einem der Ansprüche 9 bis 14,
wobei der Wellenlängen-Schwellenwert etwa zwischen 480 nm und 600 nm liegt, vorzugsweise zwischen 500 nm und 560 nm.

16. Leuchte nach einem der Ansprüche 9 bis 15,
wobei der Anteil-Schwellenwert kleiner als 10% gewählt wird, vorzugsweise kleiner als 5%.

17. Leuchte nach einem der Ansprüche 9 bis 16,
wobei das besondere Licht eine Farbtemperatur aufweist, die unterhalb eines vorab festgelegten Farbtemperatur-Schwellenwertes liegt.

18. Leuchte nach Anspruch 17,
wobei der Farbtemperatur-Schwellenwert maximal etwa 2000 K, vorzugsweise weniger als 1500 K beträgt.

## Claims

1. A method for control of a luminaire, with which light can be generated in different spectral compositions, wherein, in the control, the spectral composition of the generated light is chosen in dependence on a preset time scheme,
wherein, in a previously defined period, a special light is generated, which with respect to its spectral composition and intensity is such that, for an observer of the special light, it at least as far as possible does not inhibit release of the hormone melatonin,
wherein the special light has a spectral composition for which the following applies:
that portion (A_{BLUE}) of the intensity of the special light which is generated by radiation with wavelengths which are less than a preset wavelength threshold value is less than a preset portion threshold value (A_{S}),
wherein, in the control of the luminaire, the spectral composition of the special light is also chosen in dependence on the intensity of the special light,
**characterized in that**
the portion (A_{BLUE}) of the intensity of the special light that is generated by radiation with wavelengths less than the preset wavelength threshold value, in the case of a first intensity of the special light, is less than in the case of a second intensity of the special light, which is less than the first intensity of the special light.

2. A method according to Claim 1,
wherein to define the previously defined period the times of sunsets and/or sunrises are used.

3. A method according to Claim 1 or 2,
wherein the wavelength threshold value is approximately between 480 nm and 600 nm, preferably between 500 nm and 560 nm.

4. A method according to any one of the preceding claims, wherein the portion threshold value (A_{S}) is chosen to be less than 10%, preferably less than 5%.

5. A method according to any one of the preceding claims, wherein the special light has a colour temperature which is below a previously defined colour temperature threshold value.

6. A method according to Claim 5,
wherein the colour temperature threshold value is at most about 2000 K, preferably less than 1500 K.

7. A method according to any one of the preceding claims, wherein as the luminaire (1), a luminaire with three light sources (2, 3, 4), preferably in the form of fluorescent lamps, is used, said light sources each being able to generate light in a different colour or colour temperature, wherein the special light can be generated by one of the three light sources (2, 3, 4).

8. A method according to any one of the preceding claims, wherein the luminaire is an RGB luminaire.

9. A luminaire adapted to emit light in different spectral compositions, having
- at least two light sources, and
- a controller (5) for the light sources,
the controller (5) being adapted to control the luminaire (1) in such a way that from the luminaire (1), in a previously defined period, a special light is emitted which, with respect to its spectral composition and intensity, is such that, for an observer of the special light, it at least as far as possible does not inhibit release of the hormone melatonin,
wherein the special light has a spectral composition for which the following applies:
that portion (A_{BLUE}) of the intensity of the special light which is generated by radiation with wavelengths which are less than a preset wavelength threshold value is so small that it is below a preset portion threshold value (A_{S}),
wherein the luminaire is also adapted to emit light in different intensities,
wherein the controller (5) is also adapted to control the luminaire (1) in such a way that in the control the spectral composition of the special light is chosen in dependence on the intensity of the special light,
**characterized in that**
the controller (5) is adapted to control the luminaire (1) in such a way that the following applies for the special light:
the portion (A_{BLUE}) of the intensity of the special light which is generated by radiation with wavelengths less than the preset wavelength threshold value, in the case of a first intensity of the special light, is less than in the case of a second intensity of the special light, which is less than the first intensity of the special light.

10. A luminaire according to Claim 9,
wherein the special light is generated by one of the light sources.

11. A luminaire according to Claim 9 or 10,
wherein the light sources include fluorescent lamps.

12. A luminaire according to any one of Claims 9 to 11, wherein the luminaire has three light sources (2, 3, 4), which can radiate light in different colours or colour temperatures.

13. A luminaire according to Claim 12,
having three control channels for the three light sources (2, 3, 4).

14. A luminaire according to Claim 12 or 13,
wherein with a first (2) of the three light sources (2, 3, 4) light with a colour temperature of more than 5000 K can be radiated, and with a second (3) of the three light sources (2, 3, 4) light with a colour temperature of less than 5000 K, and with the third light source (4) the special light.

15. A luminaire according to any one of Claims 9 to 14, wherein the wavelength threshold value is approximately between 480 nm and 600 nm, preferably between 500 nm and 560 nm.

16. A luminaire according to any one of Claims 9 to 15, wherein the portion threshold value is chosen to be less than 10%, preferably less than 5%.

17. A luminaire according to any one of Claims 9 to 16, wherein the special light has a colour temperature which is below a previously defined colour temperature threshold value.

18. A luminaire according to Claim 17,
wherein the colour temperature threshold value is at most about 2000 K, preferably less than 1500 K.

## Revendications

1. Procédé pour la commande d'un luminaire, lequel permet de générer de la lumière dans différentes compositions spectrales,
dans lequel, lors de la commande, la composition spectrale de la lumière générée est choisie en fonction d'un schéma de temps prédéfini,
dans lequel, dans un laps de temps défini auparavant, on génère une lumière particulière qui est de nature, en ce qui concerne sa composition spectrale et son intensité, à ce que qu'elle n'empêche pas au moins très largement un déversement de l'hormone mélatonine chez un observateur de la lumière particulière,
dans lequel la lumière particulière présente une composition spectrale pour laquelle :
la fraction (A_{BLAU}) de l'intensité de la lumière particulière, qui est générée par du rayonnement avec des longueurs d'ondes inférieures à une valeur seuil de longueur d'onde prédéfinie, est inférieure à une valeur seuil de fraction (A_{S}) prédéfinie,
dans lequel, lors de la commande du luminaire, la composition spectrale de la lumière particulière est choisie d'autre part en fonction de l'intensité de la lumière particulière,
**caractérisé en ce que** la fraction (A_{BLAU}) de l'intensité de la lumière particulière, qui est générée par du rayonnement avec des longueurs d'onde inférieures à la valeur seuil de longueur d'onde prédéfinie, est inférieure, dans le cas d'une première intensité de la lumière particulière, par rapport au cas d'une seconde intensité de la lumière particulière, qui est inférieure à la première intensité de la lumière particulière.

2. Procédé selon la revendication 1,
dans lequel les heures de coucher du soleil et/ou de lever du soleil sont utilisées pour définir la période définie auparavant.

3. Procédé selon la revendication 1 ou 2,
dans lequel la valeur seuil de longueur d'onde se situe à peu près entre 480 nm et 600 nm, de préférence entre 500 nm et 560 nm.

4. Procédé selon l'une quelconque des revendications précédentes, dans lequel la valeur seuil de fraction (A_{S}) est choisie inférieure à 10 %, de préférence inférieure à 5 %.

5. Procédé selon l'une quelconque des revendications précédentes, dans lequel la lumière particulière présente une température de couleur qui est inférieure à une valeur seuil de température de couleur définie auparavant.

6. Procédé selon la revendication 5,
dans lequel la valeur seuil de température de couleur est au maximum d'environ 2.000 K, de préférence inférieure à 1.500 K.

7. Procédé selon l'une quelconque des revendications précédentes, dans lequel on utilise comme luminaire (1) un luminaire avec trois sources de lumière (2, 3, 4), de préférence sous forme de tubes fluorescents, qui peuvent générer chacun de la lumière dans une autre couleur ou une autre température de couleur, la lumière particulière pouvant être générée par l'une des trois sources de lumière (2, 3, 4).

8. Procédé selon l'une quelconque des revendications précédentes, dans lequel le luminaire est un luminaire RGB.

9. Luminaire, qui est conçu pour délivrer de la lumière dans différentes compositions spectrales, présentant :
- au moins deux sources de lumière, et
- un dispositif de commande (5) pour les sources de lumière,
sur lequel le dispositif de commande (5) est conçu pour commander le luminaire (1) de telle sorte que le luminaire (1) délivre dans un laps de temps défini auparavant une lumière particulière qui, en ce qui concerne sa composition spectrale et son intensité, est de nature à ne pas empêcher au moins très largement un déversement de l'hormone mélatonine chez un observateur de la lumière particulière,
sur lequel la lumière particulière présente une composition spectrale pour laquelle :
la fraction (A_{BLAU}) de l'intensité de la lumière particulière, qui est générée par du rayonnement avec des longueurs d'ondes inférieures à une valeur seuil de longueur d'onde prédéfinie, est si faible qu'elle est inférieure à une valeur seuil de fraction (A_{S}) prédéfinie,
le luminaire étant conçu également pour délivrer de la lumière dans différentes intensités,
le dispositif de commande (5) étant conçu également pour commander le luminaire (1) de telle sorte que, lors de la commande, la composition spectrale de la lumière particulière est choisie en fonction de l'intensité de la lumière particulière,
**caractérisé en ce que** le dispositif de commande (5) est conçu pour commander le luminaire (1) de telle sorte que, pour la lumière particulière :
la fraction (A_{BLAU}) de l'intensité de la lumière particulière, qui est générée par rayonnement avec des longueurs d'onde inférieures à la valeur seuil de longueur d'onde prédéfinie, est inférieure, dans le cas d'une première intensité de la lumière particulière, par rapport au cas d'une seconde intensité de la lumière particulière, qui est inférieure à la première intensité de la lumière particulière.

10. Luminaire selon la revendication 9,
sur lequel la lumière particulière est générée par l'une des sources de lumière.

11. Luminaire selon la revendication 9 ou 10,
sur lequel les sources de lumière comprennent des tubes fluorescents.

12. Luminaire selon l'une quelconque des revendications 9 à 11,
le luminaire présentant trois sources de lumière (2, 3, 4), qui peuvent diffuser de la lumière dans différentes couleurs ou températures de couleur.

13. Luminaire selon la revendication 12,
présentant trois canaux de commande pour les trois sources de lumière (2, 3, 4).

14. Luminaire selon la revendication 12 ou 13,
sur lequel de la lumière avec une température de couleur supérieure à 5.000 K peut être diffusée avec une première (2) des trois sources de lumière (2, 3, 4), et de la lumière avec une température de couleur inférieure à 5.000 K avec une seconde (3) des trois sources de lumière (2, 3, 4), et la lumière particulière avec la troisième source de lumière (4).

15. Luminaire selon l'une quelconque des revendications 9 à 14,
sur lequel la valeur seuil de longueur d'onde se situe environ entre 480 nm et 600 nm, de préférence entre 500 nm et 560 nm.

16. Procédé selon l'une quelconque des revendications 9 à 15,
sur lequel la valeur seuil de fraction est inférieure à 10 %, de préférence inférieure à 5 %.

17. Luminaire selon l'une quelconque des revendications 9 à 16,
sur lequel la lumière particulière présente une température de couleur qui est inférieure à une valeur seuil de température de couleur fixée auparavant.

18. Luminaire selon la revendication 17,
sur lequel la valeur seuil de température de couleur se situe au maximum aux environs de 2.000 K, de préférence au-dessous de 1.500 K.
